# FASCICULE DE BREVET EUROPEEN

(11) **EP 2 051 049 B1**
(45) Date de publication et mention de la délivrance du brevet: **16.08.2017**
(21) Numéro de dépôt: 08166661.2
(22) Date de dépôt: 15.10.2008
(51) Int. Cl.: G01F 23/24, G01N 27/06, F02M 37/22

(54) **Capteur de présence de liquide dans un conteneur et dispositif muni d'un tel capteur**
Flüssigkeitssensor zum Nachweis der Flüssigkeitsmenge in einem Behälter und mit einem solchen Sensor ausgerüstete Vorrichtung
Sensor for the presence of liquid in a container and device equipped with such a sensor

(30) Priorité: 15.10.2007 FR 0758332
(43) Date de publication de la demande: 22.04.2009
(73) Titulaire: G. Cartier Technologies, 74302 Cluses (FR)
(72) Inventeur: Nourdine, Amir, 74800, LA ROCHE SUR FORON (FR)
(74) Mandataire: Cabinet Poncet

(56) Documents cités:
- WO-A-03/104785
- GB-A- 1 128 839
- GB-A- 2 065 336
- GB-A- 2 254 429
- US-A1- 2007 084 283

## Description

La présente invention concerne les moyens pour détecter la présence d'un liquide dans un conteneur.

On a déjà proposé de nombreux types de capteur de présence de liquide, notamment pour contrôler le niveau de liquide dans un conteneur.

Par exemple, le document EP 0 907 070 A1 décrit un capteur de niveau de liquide pour une installation de refroidissement du moteur thermique d'un véhicule automobile. Ce capteur comprend deux électrodes parallèles qui pénètrent dans le conteneur, et un circuit électronique propre à faire circuler un signal électrique entre les électrodes de manière à réaliser un capteur de continuité. Lorsque les électrodes viennent en contact avec le liquide, le liquide ferme un circuit électrique et cette fermeture est détectée par le circuit électronique. Un tel capteur nécessite de traverser la paroi du conteneur, le circuit électronique de mesure étant à l'extérieur du conteneur, une partie au moins des électrodes étant à l'intérieur du conteneur pour être en contact avec le liquide.

La traversée de paroi du conteneur induit un risque de fuite de liquide par des interstices pouvant se produire au passage des électrodes. Ce risque n'est pas acceptable dans les conteneurs étanches, surtout les conteneurs dans lesquels l'atmosphère intérieure peut être en surpression.

Si l'on dispose le passage des électrodes en partie supérieure du conteneur, le risque de fuite est réduit, mais il subsiste tout de même dans le cas où le liquide peut remplir complètement le conteneur.

On connaît par ailleurs du document US 2007/0084283 A1 un capteur de niveau de liquide pour réservoir de produits pétroliers. Ce capteur comprend un premier élément de capteur destiné à être placé à l'extérieur du conteneur et ayant un circuit électronique à récepteur d'ondes hertziennes. D'autre part, ce capteur comprend un second élément de capteur destiné à être placé à l'intérieur du conteneur, ayant un second circuit électronique avec des moyens sensibles à la présence d'un liquide dans le conteneur, et ayant un émetteur d'ondes hertziennes pour communiquer avec le premier élément de capteur.

Les moyens sensibles à la présence d'un liquide dans le conteneur consistent en un transducteur à ultrasons, placé au-dessus du niveau du liquide, qui émet des ultrasons vers la surface du liquide et qui calcule le temps de vol des ultrasons réfléchis par le niveau de liquide.

Les données de temps de vol sont ensuite transformées en données de hauteur de niveau de liquide, qui sont transmises au premier élément de capteur par la liaison sans fil à transmission d'ondes hertziennes au moyen de l'émetteur et du récepteur d'ondes hertziennes.

On pourrait imaginer d'adapter ce dispositif à un conteneur totalement étanche, supprimant les risques de fuite, puisqu'il n'y a alors aucun élément devant traverser la paroi du conteneur.

Cependant, un tel dispositif présente plusieurs inconvénients, qui empêchent son application à certains conteneurs à faible prix et/ou étanches.

Tout d'abord, un tel capteur doit comprendre un transducteur à ultrasons, un émetteur et un récepteur d'ondes hertziennes modulées, et des moyens de calcul, ce qui implique un coût relativement élevé.

Par rapport au coût d'un réservoir de produits pétroliers, le coût de ce capteur est évidemment négligeable. Cependant, pour des applications à des réservoirs de plus petite taille ou de plus faible valeur, par exemple dans le domaine automobile, le coût de ce capteur devient prohibitif.

Un second inconvénient est la nécessité d'alimenter le second élément de capteur à l'intérieur du conteneur par une source d'énergie électrique. Le document prévoit d'incorporer une pile dans le second élément de capteur. La durée de vie des piles est limitée, et nécessite alors une intervention périodique de changement de pile, et donc l'ouverture du conteneur. Cela constitue un inconvénient important dans le cas de conteneurs dont l'accès est difficile ou dangereux, par exemple les conteneurs à grande hauteur. Et surtout cela n'est pas acceptable dans le cas de conteneurs devant rester étanches sans intervention périodique de maintenance. Tel est le cas par exemple de certains conteneurs étanches utilisés dans le domaine automobile.

On connaît aussi, du document GB 1,128,839, un capteur de niveau inférieur de liquide dans un conteneur qui comprend les moyens du préambule de la revendication 1.

Dans le dispositif décrit dans ce document, des moyens sensibles à la présence de liquide sont disposés dans le conteneur et sont connectés au secondaire d'un transformateur à circuit magnétique dont le primaire est alimenté par un oscillateur dont les oscillations sinusoïdales sont étouffées lorsque du liquide présent dans le conteneur réduit l'impédance aux bornes du secondaire du transformateur. Le dispositif nécessite la traversée de paroi du conteneur, avec les risques de fuite que cela implique.

Le problème proposé par la présente invention est de concevoir une nouvelle structure de capteur qui permette la détection de présence de liquide dans un conteneur sans traversée de paroi par des organes électriques ou autres de nature à engendrer un risque de fuite, dont la structure soit particulièrement simple et peu onéreuse, et dont le coût de production soit ainsi compatible avec des applications telles que le domaine automobile.

Selon un autre but, l'invention vise à réaliser un tel capteur dont la mise en place soit particulièrement aisée sans nécessiter des interventions de positionnement précises dans l'espace.

Selon un autre aspect, l'invention vise à éviter toute intervention de maintenance périodique sur les éléments situés à l'intérieur du conteneur, afin de garantir l'intégrité du conteneur pendant une longue durée.

Selon un autre aspect, l'invention vise à éviter les effets d'électrolyse produits par des signaux électriques alternatifs de puissance non négligeable.

Selon un autre aspect, l'invention prévoit d'appliquer un tel capteur au domaine automobile, et en particulier à la détection d'eau dans les filtres à gasoil des moteurs diesel. Cette fonction apparaît de plus en plus nécessaire avec le développement des moteurs diesel à haute pression, qui présentent un risque de destruction lors de la présence d'eau dans le gasoil.

Pour atteindre ces buts ainsi que d'autres, l'invention propose un capteur de présence de liquide pour conteneur selon la revendication 1. La liaison sans fil à transmission magnétique sans circuit magnétique continu, et l'absence de moyens d'alimentation externe du second élément de capteur, évitent toute traversée de la paroi du conteneur par des organes électriques ou autres, et évitent tout risque de fuite. Du fait que la liaison sans fil entre le premier élément de capteur et le second élément de capteur est assurée par le couplage magnétique sans circuit magnétique de deux bobines primaire et secondaire, les circuits électriques d'un tel capteur sont particulièrement simples, beaucoup plus simples que des émetteurs et récepteurs d'ondes hertziennes modulées.

En outre, un tel couplage entre deux bobines magnétiques de part et d'autre d'une paroi de conteneur peut présenter une qualité de transmission satisfaisante sans qu'il soit nécessaire d'utiliser des signaux alternatifs de forte puissance ou de placer de manière précise les bobines l'une par rapport à l'autre. Il en résulte une grande facilité d'adaptation du capteur dans un conteneur lors de la fabrication, et on limite au maximum l'effet d'électrolyse.

Selon un mode de réalisation avantageux, le capteur selon l'invention est tel que :
- l'élément à impédance variable comprend deux conducteurs électriques, connectés chacun à une borne respective du condensateur, et ayant chacun une partie conductrice apparente pour être au contact du liquide à détecter dans le conteneur, de sorte que les deux conducteurs électriques constituent, avec un liquide conducteur de l'électricité, un élément à résistance variable dont la valeur de résistance dépend de la présence et de la concentration du liquide à détecter,
- le circuit électrique analyseur compare la composante d'amortissement des signaux électriques résultants à des valeurs de référence de composante d'amortissement.

Dans ce cas, l'élément à impédance variable du capteur est une résistance électrique constituée par les deux conducteurs électriques et par la matière située dans l'intervalle entre les deux parties conductrices apparentes des conducteurs électriques. Lorsque les parties conductrices apparentes des conducteurs électriques sont dans l'air, elles sont isolées l'une de l'autre et la résistance électrique est très grande. Lorsque la matière située dans l'intervalle entre les deux parties conductrices apparentes des conducteurs électriques est un liquide électriquement isolant, la résistance est également grande. En présence d'eau dans l'intervalle entre les deux parties conductrices apparentes des conducteurs électriques, la résistance électrique est réduite, et modifie les propriétés du circuit oscillant secondaire passif. En particulier, lors d'une excitation par une tension impulsionnelle au primaire, dans le premier circuit électrique, le circuit oscillant secondaire passif entre en oscillation selon une onde sinusoïdale amortie dont l'amortissement dépend de la résistance électrique présente dans le circuit oscillant. Ainsi, la présence d'eau peut être détectée en analysant le taux d'atténuation de l'onde amortie dans le circuit oscillant secondaire passif, onde qui est transmise au premier circuit électrique à bobine primaire et qui est analysée par le circuit électrique analyseur.

On comprend qu'un tel capteur est particulièrement simple et peu onéreux, ne nécessitant aucun transducteur à ultrasons ou autre élément complexe.

Selon un mode de réalisation, pour analyser l'amortissement des signaux électriques résultants et les comparer à des valeurs de référence de composantes d'amortissement, le circuit électrique analyseur peut générer un signal qui est la dérivée des signaux électriques résultants, il peut mémoriser la suite des amplitudes des signaux électriques résultants à des instants où le signe du signal dérivé s'inverse, et il peut comparer la suite des amplitudes à une suite d'amplitudes de référence préalablement mémorisée.

L'excitation du circuit oscillant secondaire passif peut être réalisée de manière particulièrement simple en prévoyant un circuit électrique d'excitation qui comprend un élément de commutation ayant un état passant et un état bloqué, apte à commuter périodiquement en série avec la bobine primaire une source de tension continue. A chaque commutation, la bobine primaire émet une impulsion magnétique transmise à la bobine secondaire et qui excite le circuit oscillant secondaire passif.

De préférence, on considère essentiellement les signaux électriques résultants qui se produisent après l'instant où l'élément de commutation passe de son état passant vers son état bloqué. Le circuit électrique analyseur scrute alors et analyse les signaux électriques résultants, au cours de la période temporelle faisant suite à l'instant où l'élément de commutation passe de son état passant à son état bloqué. En effet, la commutation vers l'état bloqué est la configuration la plus favorable pour faciliter la détection et la mesure des paramètres des signaux électriques résultants dans le circuit électrique primaire : la bobine primaire subit une commutation rapide qui excite considérablement le circuit électrique secondaire, et simultanément la bobine primaire est déconnectée et ne subit plus l'influence de la source de tension continue, ce qui permet d'évaluer de façon plus précise les paramètres des oscillations de tension alternative amorties aux bornes de la bobine primaire.

Le circuit électrique analyseur peut avantageusement être implémenté dans un microcontrôleur qui reçoit, après conditionnement par un circuit amplificateur, la tension aux bornes de la bobine primaire. Un tel microcontrôleur peut aussi servir simultanément pour remplir d'autres fonctions intelligentes telles que le pilotage d'un élément de régulation de la température du gasoil, lorsque le capteur est appliqué au contrôle de présence d'eau dans le gasoil d'un moteur diesel.

Les commutations de l'élément de commutation génèrent sur la bobine primaire une surtension initiale de valeur très élevée, que certains composants ne supportent pas. Pour éviter les risques de dégradation de composants, on peut avantageusement prévoir, dans le premier circuit électrique, une résistance de stabilisation et un composant limiteur de tension, connectés en parallèle aux bornes de la bobine primaire.

De préférence, la bobine secondaire, le condensateur et l'élément à impédance variable sont noyés en tout ou partie dans une résine.

Selon un autre aspect, l'invention propose un conteneur à parois non métalliques, équipé d'au moins un capteur de présence de liquide tel que défini ci-dessus. Le premier élément de capteur est placé et fixé à l'extérieur du conteneur, de façon définitive ou de façon amovible. Le second élément de capteur est placé et fixé à l'intérieur du conteneur. Les bobines primaire et secondaire sont positionnées en regard l'une de l'autre de part et d'autre d'une zone intermédiaire de paroi du conteneur.

On comprend que, une fois que le second élément de capteur est placé et fixé à l'intérieur du conteneur, il n'y a plus aucun besoin d'ouvrir le conteneur. En pratique, on peut prévoir une paroi étanche de conteneur, munie d'un orifice d'insertion permettant l'introduction du second élément de capteur, et obturable par un bouchon. Après fermeture, il n'y a plus aucun besoin d'ouvrir le conteneur pour le fonctionnement ou la maintenance du capteur selon l'invention, et le conteneur peut donc rester étanche pendant toute son utilisation.

De préférence, les bobines primaire et secondaire sont disposées sensiblement coaxiales et au plus près l'une de l'autre de part et d'autre de la zone intermédiaire de paroi.

Dans le cas d'une fixation amovible du premier élément de capteur, ce dernier constitue un élément portable permettant alors de vérifier successivement plusieurs conteneurs.

Selon un autre aspect, l'invention propose une application aux filtres à gasoil pour moteur diesel, le conteneur constituant alors l'enveloppe d'un filtre à gasoil pour moteur diesel, le capteur de présence de liquide selon l'invention étant agencé pour détecter la présence d'eau dans le gasoil à l'intérieur du filtre à gasoil.

Le signal de sortie produit par le capteur de présence de liquide selon l'invention peut être avantageusement utilisé pour commander une électrovanne de purge apte à extraire l'eau du gasoil.

D'autres objets, caractéristiques et avantages de la présente invention ressortiront de la description suivante de modes de réalisation particuliers, faite en relation avec les figures jointes, parmi lesquelles :
- la figure 1 est une vue schématique illustrant la structure générale d'un capteur de présence de liquide dans un conteneur étanche selon un mode de réalisation de la présente invention ;
- la figure 2 illustre le schéma électrique de principe du capteur selon un mode de réalisation de la présente invention ;
- la figure 3 est un diagramme temporel illustrant la forme d'onde (amplitude) des signaux électriques résultants dans le cas d'une absence de détection de liquide ; et
- la figure 4 est un diagramme temporel illustrant la forme d'onde (amplitude) des signaux électriques résultants dans le cas d'une détection de liquide conducteur de l'électricité.

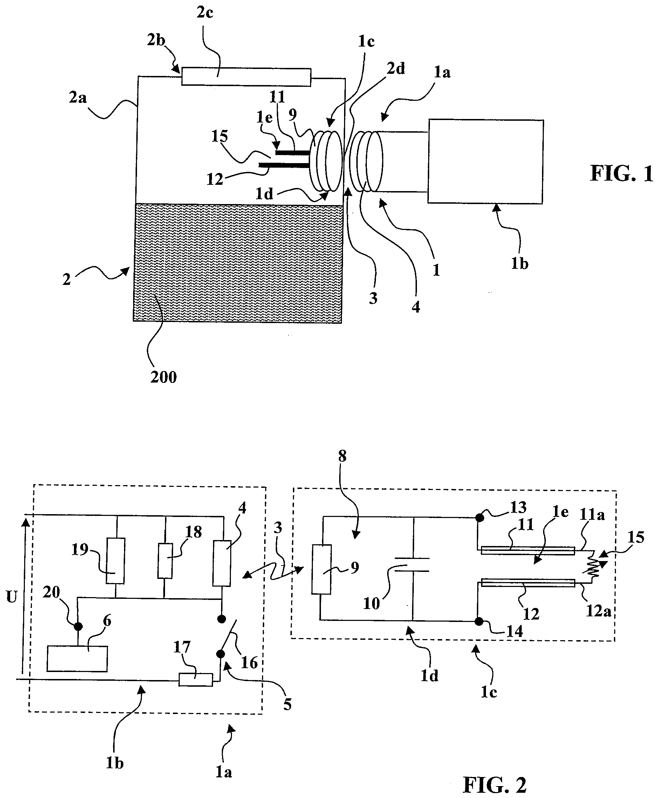

Dans le mode de réalisation illustré sur la figure 1, le capteur 1 est associé à un conteneur étanche 2.

Le capteur 1 comprend un premier élément de capteur 1 a que l'on place à l'extérieur du conteneur 2, et qui comprend un premier circuit électrique 1 b. Un second élément de capteur 1 c est placé à l'intérieur du conteneur 2, et comprend un second circuit électrique 1 d et des moyens 1 e sensibles à la présence d'un liquide 200 dans le conteneur 2. Une liaison sans fil 3 par induction magnétique sans circuit magnétique est prévue pour transmettre des signaux électriques entre le premier circuit électrique 1 b et le second circuit électrique 1 d, assurant la transmission de signaux de présence de liquide émis par le second circuit électrique 1d.

On distingue, sur la figure 1, que le premier circuit électrique 1b comprend une bobine primaire 4, tandis que le second circuit électrique 1 d comprend une bobine secondaire 9. La liaison sans fil 3 entre le premier élément de capteur 1a et le second élément de capteur 1 c est assurée par le couplage magnétique entre les bobines primaire 4 et secondaire 9. Le couplage s'effectue à travers la paroi du conteneur 2, sans l'aide d'un circuit magnétique continu ou fermé, à matériau ferromagnétique tel que ceux des transformateurs.

Le conteneur 2 est formée d'une paroi périphérique 2a munie d'un orifice d'insertion 2b obturable par un bouchon 2c. Le bouchon 2c est ouvert lors de l'assemblage, pour l'introduction du second élément de capteur 1 c à l'intérieur du conteneur 2 et sa fixation sur la face interne de la paroi 2a, contre une zone intermédiaire 2d de la paroi 2a du conteneur 2. Le bouchon 2c est ensuite refermé, et peut être scellé, aucune ouverture n'étant nécessaire ultérieurement.

On considère maintenant la figure 2.

On retrouve le premier élément de capteur 1 a et le second élément de capteur 1 c ayant respectivement un premier circuit électrique 1 b et un second circuit électrique 1 d.

Le premier circuit électrique 1 b comprend la bobine primaire 4, un circuit électrique d'excitation 5 apte à générer sur la bobine primaire 4 des tensions impulsionnelles d'excitation, et un circuit électrique analyseur 6.

Le second circuit électrique 1 d est un circuit oscillant secondaire passif 8, comprenant la bobine secondaire 9 et un condensateur 10. Le moyen sensible à la présence d'un liquide 200 dans le conteneur 2 est un élément à impédance variable 1 e dont l'impédance varie selon que du liquide 200 est présent ou absent dans le conteneur 2 et dont la variation d'impédance modifie les propriétés d'oscillation du circuit oscillant secondaire passif 8.

Dans le mode de réalisation illustré sur les figures, l'élément à impédance variable 1 e comprend deux conducteurs électriques 11 et 12, connectés chacun à une borne respective 13 ou 14 du condensateur 10, et ayant chacun une partie conductrice apparente 11 a ou 12a pour être au contact du liquide 200 dont la présence doit être recherchée dans le conteneur 2. Les deux parties conductrices apparentes 11 a et 12a sont séparées par un intervalle 15 pouvant être occupé par du liquide 200. En présence d'un liquide 200 conducteur de l'électricité dans l'intervalle 15, les deux conducteurs électriques 11 et 12 constituent, avec le liquide 200 dans l'intervalle 15, un élément résistif dont la valeur de résistance affecte les propriétés du circuit oscillant secondaire passif 8.

Le circuit électrique analyseur 6 peut donc être agencé de manière à détecter l'influence de cette résistance sur les oscillations du circuit oscillant secondaire passif 8, afin de détecter la présence de liquide 200 entre les deux parties conductrices apparentes 11 a et 12a de l'élément à impédance variable 1 e.

Le circuit électrique d'excitation 5 comprend un élément de commutation 16, tel qu'un transistor piloté par le circuit électrique analyseur 6, et qui présente un état passant et un état bloqué. L'élément de commutation 16 est connecté de façon à commuter périodiquement en série avec la bobine primaire 4 une source de tension continue U. Par exemple, dans les applications au domaine automobile, la source de tension continue peut être la batterie du véhicule.

Une résistance 17 en série limite le courant électrique s'établissant dans la bobine primaire 4 lorsque l'élément de commutation 16 est à l'état passant.

Une résistance de stabilisation 18 et un composant limiteur de tension 19 sont connectés en parallèle aux bornes de la bobine primaire 4. Le composant limiteur de tension 19 peut être par exemple un composant à base de diodes Zener.

On considère maintenant le fonctionnement du circuit électrique de la figure 2.

Initialement, on commute l'élément de commutation 16 à l'état passant. Un courant électrique s'établit alors dans la bobine primaire 4, et devient rapidement continu et de valeur déterminée par la résistance 17.

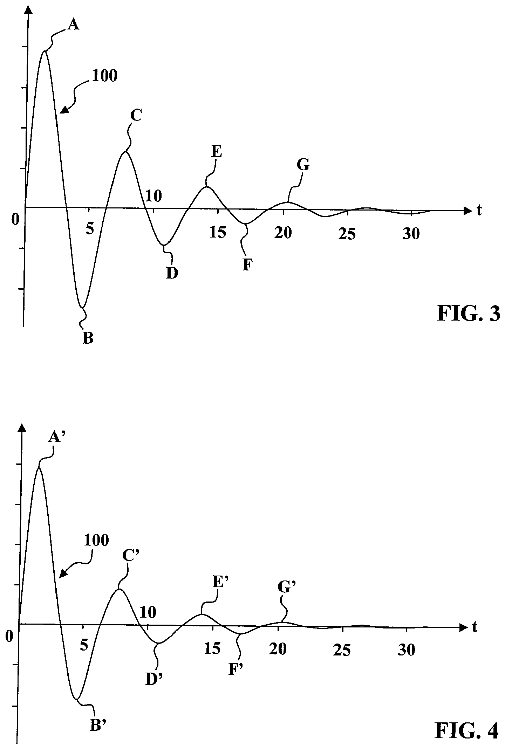

On commute alors l'élément de commutation 16 à l'état bloqué, réalisant une déconnexion brusque de la source de tension continue U aux bornes de la bobine primaire 4. Cette déconnexion provoque la production, aux bornes de la bobine primaire 4, d'un échelon de tension de forte valeur, écrêté par le composant limiteur de tension 19. Cet échelon de tension, ou variation très brusque de tension, génère une variation rapide du champ magnétique créé par la bobine primaire 4 et qui se transmet à la bobine secondaire 9 à travers la paroi 2a (figure 1) du conteneur 2, laquelle paroi 2a étant en matériau non métallique. L'impulsion reçue par la bobine secondaire 9 excite le circuit oscillant secondaire passif 8, dans lequel apparaît une oscillation électrique sinusoïdale amortie dont la forme (amplitude de tension) est similaire à celle illustrée sur la figure 3.

Cette oscillation sinusoïdale amortie est transmise de la bobine secondaire 9 vers la bobine primaire 4 et se propage dans le premier circuit électrique 1 b sous forme de signaux électriques résultants 100 qui comportent une composante à onde sinusoïdale amortie telle qu'illustrée sur la figure 3.

Ainsi, les signaux électriques résultants 100 apparaissant dans le premier circuit électrique 1 b sont l'image des signaux d'oscillation apparaissant dans le circuit oscillant secondaire passif 8.

En l'absence d'un liquide 200 conducteur de l'électricité dans l'intervalle 15 entre les parties conductrices apparentes 11a et 12a des conducteurs électriques 11 et 12, les oscillations du circuit oscillant secondaire passif 8 sont relativement peu amorties, comme illustré sur la figure 3.

Par contre, en présence d'un liquide 200 conducteur de l'électricité dans l'intervalle 15, la conduction électrique possible dans l'intervalle 15 amortit plus rapidement les oscillations du circuit oscillant secondaire passif 8, de sorte que les signaux électriques résultants 100 deviennent plus rapidement amortis, comme illustré sur la figure 4.

Le circuit électrique analyseur 6 est programmé pour scruter et analyser les signaux électriques résultants 100 au cours de la période temporelle qui fait suite à l'instant où l'élément de commutation 16 passe de son état passant vers son état bloqué. Le circuit électrique analyseur 6 est pour cela implémenté dans un microcontrôleur qui reçoit, après conditionnement par un circuit amplificateur non illustré sur les figures, la tension aux bornes de la bobine primaire, ou tension prise au point de mesure 20.

Comme déjà indiqué, cette tension comporte une composante sinusoïdale amortie telle qu'illustrée sur les figures 3 et 4.

Le circuit électrique analyseur 6 doit donc, dans ce mode de réalisation de l'élément à impédance variable 1 e, comparer l'amortissement des signaux électriques résultants 100 qui se produisent en présence de liquide 200 avec l'amortissement qui se produit en l'absence de liquide 200.

En d'autres termes, le circuit électrique analyseur 6 doit comparer les courbes des figures 4 et 3.

Pour cela, dans un mode de réalisation, au cours d'une étape de mémorisation préalable, on fait fonctionner le capteur selon l'invention en l'absence de liquide à détecter. Des signaux électriques résultants 100 relativement peu amortis, tels qu'illustrés sur la figure 3, se produisent aux bornes de la bobine primaire 4. Le circuit électrique analyseur 6 peut générer un signal qui est la dérivée des signaux électriques résultants 100, et il mémorise la suite des amplitudes des signaux électriques résultants 100 à des instants où le signe du signal dérivé s'inverse. Autrement dit, le circuit électrique analyseur mémorise les amplitudes du signal résultant de la figure 3 aux instants repérés par les références A, B, C, D, E, F et G par exemple. Cette suite d'amplitudes A, B, C, D, E, F et G, enregistrée dans une mémoire du circuit électrique analyseur 6, constitue les paramètres de référence qui permettront ensuite, par comparaison, de déterminer si un liquide 200 conducteur est présent dans le conteneur 2.

Ensuite, le circuit électrique analyseur 6 peut être utilisé pour la détection de présence de liquide 200. En présence de liquide 200, les signaux électriques résultants 100 ont l'allure illustrée sur la figure 4, et la même procédure que celle précédemment utilisée permet de mémoriser la suite des amplitudes des signaux électriques résultants 100 aux instants A', B', C', D', E', F' et G'. Le circuit électrique analyseur 6 peut ensuite comparer les deux suites d'amplitudes. Dans le cas d'une quasi égalité, le circuit électrique analyseur 6 en déduit l'absence de liquide 200. En cas de différence, le circuit électrique analyseur 6 peut émettre un signal indiquant la présence d'un liquide 200.

En prévoyant un circuit électrique analyseur 6 qui peut détecter avec une bonne sensibilité les différences de paramètres d'amortissement dans les signaux électriques résultants 100, on peut aussi imaginer d'apprécier la concentration de liquide 200 conducteur de l'électricité dans le conteneur 2, dans le cas où la résistance électrique du milieu dans l'intervalle 15 varie de façon sensible avec cette concentration.

En considérant à nouveau la figure 1, on voit que les bobines primaire 4 et secondaire 9 sont disposées en regard l'une de l'autre de part et d'autre de la zone intermédiaire 2d de la paroi 2a du conteneur 2. Il est seulement nécessaire que la zone intermédiaire 2d de la paroi 2a du conteneur 2 soit réalisée en un matériau laissant passer le champ magnétique entre les bobines primaire 4 et secondaire 9. Le reste de la paroi 2a du conteneur 2 peut être réalisé dans le même matériau, ou dans un autre matériau, pour autant que sa constitution ne perturbe pas la transmission du champ magnétique entre les deux bobines 4 et 9.

De préférence, les bobines primaire 4 et secondaire 9 sont disposées sensiblement coaxiales l'une par rapport à l'autre, et au plus près l'une de l'autre de part et d'autre de la zone intermédiaire 2d de la paroi 2a.

Les conducteurs électriques 11 et 12 de l'élément à impédance variable 1 e peuvent être orientés perpendiculairement par rapport au plan de la bobine secondaire 9 comme illustré sur la figure 1. En alternative, ces conducteurs peuvent être orientés différemment, par exemple parallèlement au plan de la bobine secondaire 9, ou dans toute autre orientation oblique.

On place les bobines primaire 4 et secondaire 9 de telle façon que les conducteurs électriques de l'élément à impédance variable 1 e se trouve dans la zone du conteneur 2 dans laquelle on veut détecter la présence d'un liquide 200.

Selon une application importante de l'invention, le capteur 1 de présence de liquide est implanté dans l'organe de filtrage et de réchauffage du gasoil d'un moteur diesel. Dans ce cas, le conteneur 2 est étanche et est l'organe de filtrage et de réchauffage lui-même. Le premier élément de capteur 1 a est placé à l'extérieur, et n'est donc pas en contact avec le gasoil. Le second élément de capteur 1 c est placé à l'intérieur du conteneur, et vient donc en contact avec le gasoil. Le capteur a dans ce cas pour but de détecter la présence d'eau dans le gasoil.

Le signal de sortie généré par le capteur 1 selon la présente invention peut alors avantageusement permettre de commander une électrovanne de purge, apte à extraire l'eau détectée dans le gasoil.

Une difficulté peut résulter de l'utilisation du microcontrôleur, qui est un élément susceptible d'augmenter le coût de production du dispositif. Dans ce cas, le microcontrôleur peut être agencé pour remplir d'autres fonctions intelligentes qui sont nécessaires dans le même environnement du capteur 1 et du conteneur étanche 2. Par exemple, le microcontrôleur peut piloter la vanne de purge, il peut aussi piloter les moyens de régulation de la température du gasoil, et il peut piloter d'autres éléments d'un véhicule.

La température du gasoil peut être régulée en utilisant par exemple un capteur de température qui donne une information au microcontrôleur, lequel pilote des éléments chauffants immergés dans l'environnement du filtre. Les connecteurs et le câblage sont ainsi simplifiés.

La présente invention n'est pas limitée aux modes de réalisation qui ont été explicitement décrits, mais elle en inclut les diverses variantes et généralisations contenues dans le domaine des revendications ci-après.

## Revendications

1. - Capteur (1) de présence de liquide pour conteneur (2), comprenant :
- un premier élément de capteur (1 a), adapté pour être placé à l'extérieur du conteneur (2), et ayant un premier circuit électrique (1b) comprenant une bobine primaire (4),
- un second élément de capteur (1c) ayant un second circuit électrique (1d) avec une bobine secondaire (9) et avec des moyens (1e) sensibles à la présence d'un liquide (200) dans le conteneur (2),
- un couplage magnétique (3) entre les bobines primaire (4) et secondaire (9) des premier circuit électrique (1b) et second circuit électrique (1d), pour transmettre au premier circuit électrique (1b) des signaux de présence du liquide émis par le second circuit électrique (1 d),
- le second circuit électrique (1d) est un circuit oscillant secondaire passif (8), comprenant la bobine secondaire (9) et un condensateur (10), avec un élément à impédance variable (1 e), dont l'impédance varie selon que le liquide (200) est présent ou absent, et dont la variation d'impédance modifie les propriétés d'oscillation du circuit oscillant secondaire passif (8),
**caractérisé en ce que** :
- le second élément de capteur (1c) est adapté pour être placé à l'intérieur du conteneur (2),
- le couplage magnétique (3) entre les bobines primaire (4) et secondaire (9) des premier élément de capteur (1a) et second élément de capteur (1c) est sans circuit magnétique continu ou fermé à matériau ferromagnétique,
- le premier circuit électrique (1b) comprend un circuit électrique d'excitation (5) apte à générer sur la bobine primaire (4) des tensions impulsionnelles d'excitation, et un circuit électrique analyseur (6),
- au cours d'une séquence d'analyse, le circuit électrique analyseur (6) scrute les signaux électriques résultants (100) qui apparaissent sur la bobine primaire (4) à la suite de l'application d'une tension impulsionnelle d'excitation, compare des paramètres de ces signaux électriques résultants (100) à des paramètres de référence, et génère un signal de sortie fonction du résultat de cette comparaison.

2. - Capteur selon la revendication 1, **caractérisé en ce que** :
- l'élément à impédance variable (1e) comprend deux conducteurs électriques (11, 12), connectés chacun à une borne respective (13, 14) du condensateur (10), et ayant chacun une partie conductrice apparente (11a, 12a) pour être au contact du liquide (200) à détecter dans le conteneur (2), de sorte que les deux conducteurs électriques (11, 12) constituent, avec un liquide (200) conducteur de l'électricité, un élément à résistance variable dont la valeur de résistance dépend de la présence et de la concentration du liquide (200) à détecter,
- le circuit électrique analyseur (6) compare la composante d'amortissement des signaux électriques résultants (100) à des valeurs de référence de composante d'amortissement.

3. - Capteur selon la revendication 2, **caractérisé en ce que** le circuit électrique analyseur (6) génère un signal qui est la dérivée des signaux électriques résultants (100), mémorise la suite des amplitudes (A', B', C', D', E', F', G') des signaux électriques résultants (100) à des instants où le signe du signal dérivé s'inverse, et compare la suite des amplitudes (A', B', C', D', E', F', G') à une suite d'amplitudes de référence (A, B, C, D, E, F, G) préalablement mémorisée.

4. - Capteur selon l'une quelconque des revendications 1 à 3, **caractérisé en ce que** le circuit électrique d'excitation (5) comprend un élément de commutation (16) ayant un état passant et un état bloqué, apte à commuter périodiquement en série avec la bobine primaire (4) une source de tension continue (U).

5. - Capteur selon la revendication 3, **caractérisé en ce que** le circuit électrique analyseur (6) scrute et analyse les signaux électriques résultants (100) au cours de la période temporelle faisant suite à l'instant où l'élément de commutation (16) passe de son état passant vers son état bloqué.

6. - Capteur selon l'une quelconque des revendications 1 à 5, **caractérisé en ce que** le circuit électrique analyseur (6) est implémenté dans un microcontrôleur recevant, après conditionnement par un circuit amplificateur, la tension aux bornes (20) de la bobine primaire (4).

7. - Capteur selon l'une quelconque des revendications 1 à 6, **caractérisé en ce que** le premier circuit électrique (1b) comprend une résistance de stabilisation (18) et un composant limiteur de tension (19) aux bornes de la bobine primaire (4).

8. - Capteur selon l'une quelconque des revendications 1 à 7, **caractérisé en ce que** la bobine secondaire (9), le condensateur (10) et l'élément à impédance variable (1 e) sont noyés en tout ou partie dans une résine.

9. - Conteneur (2) à parois non métalliques, équipé d'au moins un capteur de présence de liquide (1) selon l'une quelconque des revendications 1 à 8, dans lequel le premier élément de capteur (1 a) est placé et fixé à l'extérieur du conteneur (2) et le second élément de capteur (1c) est placé et fixé entièrement à l'intérieur du conteneur (2), les bobines primaire (4) et secondaire (9) étant positionnées en regard l'une de l'autre de part et d'autre d'une zone intermédiaire (2d) de la paroi (2a) du conteneur (2).

10. - Conteneur selon la revendication 9, **caractérisé en ce que** les bobines primaire (4) et secondaire (9) sont disposées sensiblement coaxiales et au plus près l'une de l'autre de part et d'autre de la zone intermédiaire (2d) de la paroi (2a).

11. - Conteneur selon l'une des revendications 9 ou 10, **caractérisé en ce qu'**il comprend un orifice d'insertion du second élément de capteur qui, après positionnement et fixation du second élément de capteur (1c), est obturé de façon étanche pour constituer un conteneur étanche (2).

12. - Conteneur selon l'une quelconque des revendications 9 à 11, **caractérisé en ce qu'**il constitue un filtre à gasoil pour moteur diesel, le capteur de présence de liquide (1) étant agencé pour détecter la présence d'eau dans le gasoil à l'intérieur du filtre à gasoil.

13. - Conteneur selon la revendication 12, **caractérisé en ce que** le signal de sortie commande une électrovanne de purge apte à extraire l'eau du gasoil.

14. - Conteneur selon l'une des revendications 12 ou 13, **caractérisé en ce que** le circuit électrique analyseur (6) est implémenté dans un microcontrôleur qui remplit d'autres fonctions intelligentes tel que le pilotage d'éléments de régulation de la température du gasoil.

## Patentansprüche

1. - Flüssigkeitssensor (1) für Container (2), umfassend:
- ein erstes Sensorelement (1a) zum Anordnen an einer Außenseite des Containers (2), das einen ersten elektrischen Schaltkreis (1b) mit einer Primärspule (4) besitzt,
- ein zweites Sensorelement (1c), das einen zweiten elektrischen Schaltkreis (1d) mit einer Sekundärspule (9) und mit Mitteln (1e) zum Erfassen von in dem Container (2) vorhandener Flüssigkeit (200) besitzt,
- eine magnetische Kopplung (3) zwischen der Primärspule (4) und der Sekundärspule (9) des ersten elektrischen Schaltkreises (1b) und des zweiten elektrischen Schaltkreises (1d) zum Übertragen von vorhandener Flüssigkeit anzeigenden Signalen vom ersten elektrischen Schaltkreis (1b) an den zweiten elektrischen Schaltkreis (1d),
- wobei der zweite elektrische Schaltkreis (1d) eine die Sekundärspule (9) und einen Kondensator (10) umfassende passive zweite Oszillatorschaltung (8) mit einem Element (1e) variabler Impedanz ist, dessen Impedanz in Abhängigkeit davon variiert, ob die Flüssigkeit (200) vorhanden oder nicht vorhanden ist, und dessen Variation der Impedanz die Eigenschaften der passiven zweiten Oszillatorschaltung (8) modifiziert,
**dadurch gekennzeichnet, dass**:
- das zweite Sensorelement (1c) eingerichtet ist, im Inneren des Containers (2) platziert zu werden,
- die magnetische Kopplung (3) zwischen der Primärspule (4) und der Sekundärspule (9) des ersten Sensorelementes (1a) und zweiten Sensorelementes (1c) frei von einer kontinuierlichen oder geschlossenen magnetischen Schaltung mit ferromagnetischem Material sind,
- das erste Sensorelement (1b) eine elektrische Erregerschaltung (5), die eingerichtet ist, über der Primärspule (4) Erregerspannungspulse zu generieren und eine elektrische Auswerteschaltung (6) umfasst,
- während einer Analysesequenz die elektrische Auswerteschaltung (6) die elektrischen Antwortsignale (100) prüft, die über der Primärspule (4) in Folge des Anlegens der Erregerspannungspulse auftreten, Parameter der elektrischen Antwortsignale (100) und Referenzparameter vergleicht und ein Ausgangsfunktionssignal als Ergebnis des Vergleichs generiert.

2. - Sensor nach Anspruch 1, **dadurch gekennzeichnet, dass**:
- das Element variabler Impedanz (1e) zwei elektrische Leiter (11, 12) umfasst, von denen jeder an einen entsprechenden Anschluss (13, 14) des Kondensators (10) angeschlossen ist und von denen jeder einen freigelegten leitfähigen Teil (11a, 12a) besitzt, der in Kontakt mit der im Container (2) zu detektierenden Flüssigkeit (200) bringbar ist, so dass die zwei elektrischen Leiter (11, 12) mit einer elektrisch leitenden Flüssigkeit (200) ein variables Widerstandselement bilden, dessen Widerstandswert von der Anwesenheit und der Konzentration der zu detektierenden Flüssigkeit (200) abhängt,
- wobei die elektrische Auswerteschaltung (6) die Dämpfungskomponente des elektrischen Antwortsignals (100) mit einem Referenzwert für die Dämpfungskomponente vergleicht.

3. - Sensor nach Anspruch 2, **dadurch gekennzeichnet, dass** die Auswerteschaltung (6) ein Signal erzeugt, welches die Ableitung aus den elektrischen Antwortsignalen (100) ist, die Folge von Amplituden (A', B', C', D', E', F', G') der elektrischen Antwortsignale (100) zu Augenblicken, in denen sich das abgeleitete Signal invertiert und die Folge der Amplituden (A', B', C', D', E', F', G') mit einer Folge von vorgespeicherten Referenzamplituden (A, B, C, D, E, F, G) vergleicht.

4. - Sensor nach einem der Ansprüche 1 bis 3, **dadurch gekennzeichnet, dass** die elektrische Erregerschaltung (5) ein einen Durchlasszustand und einen Blockierzustand besitzendes Kommutierungselement (16) umfasst, welches eingerichtet ist, aufeinanderfolgend periodisch eine Gleichspannungsquelle (U) auf die Primärspule (4) aufzuschalten.

5. - Sensor nach Anspruch 3, **dadurch gekennzeichnet, dass** die elektrische Auswerteschaltung (6) die elektrischen Antwortsignale (100) prüft und analysiert, in der Zeit folgend auf den Augenblick, in dem das Kommutierungselement (16) übergeht von seinem durchlassenden Zustand zu seinem blockierenden Zustand.

6. - Sensor nach einem der Ansprüche 1 bis 5, **dadurch gekennzeichnet, dass** die elektrische Auswerteschaltung (6) als Mikrocontroller implementiert ist, die, verarbeitet durch eine Verstärkerschaltung, die Klemmenspannung (20) der Primarspule (4) empfängt.

7. - Sensor nach einem der Ansprüche 1 bis 6, **dadurch gekennzeichnet, dass** der erste elektrische Schaltkreis (1b) einen Stabilisierungswiderstand (18) und ein Spannungsbegrenzungsbauteil (19) an den Klemmen der Primärspule (4) umfasst.

8. - Sensor nach einem der Ansprüche 1 bis 7, **dadurch gekennzeichnet, dass** die Sekundärspule (9), der Kondensator (10) und das Element (1e) variabler Impedanz ganz oder teilweise in einem Harz eingegossen sind.

9. - Container (2) mit metallischen Wänden, ausgestattet mit wenigstens einem Flüssigkeitssensor (1) nach einem der Ansprüche 1 bis 8, in dem das erste Sensorelement (1a) außerhalb des Containers (2) platziert und fixiert und das zweite Sensorelement (1c) vollständig im Inneren des Containers (2) platziert ist, wobei die Primärspule (4) und die Sekundärspule (9) gegenseitig gesehen an beiden Seiten einer Zwischenzone (2d) der Wand (2a) des Containers (2) platziert sind.

10. - Container nach Anspruch 9, **dadurch gekennzeichnet, dass** die Primärspule (4) und Sekundärspule (9) im Wesentlichen koaxial und möglichst nahe aneinander an beiden Seiten der Zwischenzone (2d) der Wand (2a) angeordnet sind.

11. - Container nach Anspruch 9 oder 10, **dadurch gekennzeichnet, dass** er eine Öffnung zum Einführen des zweiten Sensorelementes umfasst, welche nach dem Positionieren und Fixieren des zweiten Sensorelementes (1c) feuchtigkeitssicher abgedichtet wird, um einen feuchtigkeitssicheren Container (2) zu bilden.

12. - Container nach einem der Ansprüche 9 bis 11, **dadurch gekennzeichnet, dass** er einen Dieselfilter für Dieselfahrzeuge bildet, wobei der Flüssigkeitssensor (1) eingerichtet ist, vorhandenes Wasser im Diesel im Inneren des Dieselfilters zu detektieren.

13. - Container nach Anspruch 12, **dadurch gekennzeichnet, dass** das Ausgangssignal ein Entleerungsmagnetventil steuert, das eingerichtet ist, das Wasser aus dem Diesel zu entfernen.

14. - Container nach einem der Ansprüche 12 oder 13, **dadurch gekennzeichnet, dass** die Auswerteschaltung (6) in einem Mikrocontroller implementiert ist, der andere intelligente Funktionen erfüllt, wie zum Beispiel die Steuerung von Elementen zur Regulierung der Temperatur des Diesel.

## Claims

1. Liquid-presence sensor (1) for a container (2), said sensor comprising:
- a first sensing element (1a), suitable for being placed outside the container (2), and having a first electrical circuit (1b) comprising a primary coil (4),
- a second sensing element (1c) having a second electrical circuit (1d) with a secondary coil (9) and with means (1e) that are able to sense the presence of a liquid (200) in the container (2),
- a magnetic coupling (3) between the primary coil (4) and secondary coil (9) of the first electrical circuit (1b) and the second electrical circuit (1d), for transmitting to the first electrical circuit (1b) liquid-presence signals emitted by the second electrical circuit (1d),
- the second electrical circuit (1d) being a passive secondary oscillating circuit (8), comprising the secondary coil (9) and a capacitor (10), with a variable-impedance element (1e), the impedance of which varies depending on whether the liquid (200) is present or absent, and the impedance variation of which modifies the oscillation properties of the passive secondary oscillating circuit (8),
**characterized in that**:
- the second sensing element (1c) is suitable for being placed inside the container (2),
- the magnetic coupling (3) between the primary coil (4) and secondary coil (9) of the first sensing element (1a) and second sensing element (1c) is without a continuous or closed magnetic circuit containing ferromagnetic material,
- the first electrical circuit (1b) comprises an exciting electrical circuit (5) able to generate in the primary coil (4) exciting voltage pulses, and an analysing electrical circuit (6),
- during an analysing sequence, the analysing electrical circuit (6) scrutinizes the resulting electrical signals (100) that appear on the primary coil (4) following the application of an exciting voltage pulse, compares parameters of these resulting electrical signals (100) to reference parameters, and generates an output signal dependent on the result of this comparison.

2. Sensor according to claim 1, **characterized in that**:
- the variable-impedance element (1e) comprises two electrical conductors (11, 12) that are each connected to a respective terminal (13, 14) of the capacitor (10), and that each have an apparent conductive portion (11a, 12a) for making contact with the liquid (200) to be detected in the container (2), so that the two electrical conductors (11, 12) form, with an electrically conductive liquid (200), a variable-resistance element the resistance of which depends on the presence and the concentration of the liquid (200) to be detected,
- the analysing electrical circuit (6) compares the damping component of the resulting electrical signals (100) to reference damping-component values.

3. Sensor according to claim 2, **characterized in that** the analysing electrical circuit (6) generates a signal that is the derivative of the resulting electrical signals (100), stores in memory the sequence of amplitudes (A', B', C', D', E', F', G') of the resulting electrical signals (100) at instants at which the sign of the derivative signal changes, and compares the sequence of amplitudes (A', B', C', D', E', F', G') to a sequence of reference amplitudes (A, B, C, D, E, F, G) stored in memory beforehand.

4. Sensor according to any one of claims 1 to 3, **characterized in that** the exciting electrical circuit (5) comprises a switching element (16) that has an on state and an off state, that is able to connect periodically in series with the primary coil (4) a DC voltage source (U).

5. Sensor according to claim 3, **characterized in that** the analysing electrical circuit (6) scrutinizes and analyses the resulting electrical signals (100) during the time period following the instant at which the switching element (16) passes from its on state to its off state.

6. Sensor according to any one of claims 1 to 5, **characterized in that** the analysing electrical circuit (6) is implemented in a microcontroller receiving, after conditioning by an amplifying circuit, the voltage across the terminals (20) of the primary coil (4).

7. Sensor according to any one of claims 1 to 6, **characterized in that** the first electrical circuit (1b) comprises a stabilizing resistor (18) and a component (19) for limiting the voltage across the terminals of the primary coil (4).

8. Sensor according to any one of claims 1 to 7, **characterized in that** the secondary coil (9), the capacitor (10) and the variable-impedance element (1e) are entirely or partially embedded in a resin.

9. Container (2) with non-metal walls, said container being equipped with at least one liquid-presence sensor (1) according to any one of claims 1 to 8, wherein the first sensing element (1a) is placed on and fastened to the exterior of the container (2) and the second sensing element (1c) is placed and fastened entirely in the interior of the container (2), the primary coil (4) and secondary coil (9) being positioned facing each other on either side of an intermediate zone (2d) of the wall (2a) of the container (2).

10. Container according to claim 9, **characterized in that** the primary coil (4) and secondary coil (9) are placed substantially coaxially and as close as possible to each other on either side of the intermediate zone (2d) of the wall (2a).

11. Container according to either of claims 9 and 10, **characterized in that** it comprises an orifice for inserting the second sensing element which, after the second sensing element (1c) has been positioned and fastened in place, is seal-tightly blocked in order to form a seal-tight container (2).

12. Container according to any one of claims 9 to 11, **characterized in that** it is a diesel filter for a diesel engine, the liquid-presence sensor (1) being arranged to detect the presence of water in the diesel in the interior of the diesel filter.

13. Container according to claim 12, **characterized in that** the output signal controls a purge solenoid valve able to extract the water from the diesel.

14. Container according to either of claims 12 and 13, **characterized in that** the analysing electrical circuit (6) is implemented in a microcontroller that provides other smart functions such as the function of driving elements for regulating the temperature of the diesel.
